# EUROPEAN PATENT APPLICATION

(11) **EP 4 344 653 A1**
(43) Date of publication of application: **03.04.2024**
(21) Application number: 22198073.3
(22) Date of filing: 27.09.2022
(51) Int. Cl.: A61B 8/12, A61B 8/00, B06B 1/06

(54) **ULTRASOUND ENDOSCOPY**

(71) Applicant: Ambu A/S, 2750 Ballerup (DK)
(72) Inventor: DIEDERICHSEN, Søren Elmin, 2750 Ballerup (DK); SONNENBORG, Finn, 3600 Frederikssund (DK); SØRENSEN, Morten, 2750 Ballerup (DK)
(74) Representative: AWA Denmark A/S

(57) **Abstract**

Disclosed is an ultrasound transducer (100) comprising a convex ultrasound transducer array comprising a plurality of ultrasound transducer elements (180), an electrical circuit for receiving and providing electrical signals to the plurality of ultrasound transducer elements (180), and a support structure (101) having a convex upper surface (102) facing the plurality of ultrasound transducer elements (180) and extending in a direction parallel to a first axis (191). The electric circuit comprises a plurality of first flexible electrical conductors (150-162) bend around the support structure (101) at a first curve section (140) and at a second curve section (141), the first curve section (140) being separate from the second curve section (141).

## Description

### Field

The present disclosure relates to an ultrasound transducer, a printed flexible electrical circuit, an endoscope comprising an ultrasound transducer, and an endoscope system.

### Background

Endoscopes are widely used in hospitals for visually examining body cavities and obtaining samples of tissue identified as potentially pathological. An endoscope typically comprises an image capturing device arranged at the distal end of the endoscope either looking forward or to the side.

The image capturing device is however restricted by the limited penetration depth of visible light.

To examining deeper structures other imaging modalities must be used.

One possibility is to provide the endoscope with a curvilinear ultrasound transducer. By using emission of ultrasound waves deeper structures may be examined.

A modern curvilinear ultrasound transducer comprises a plurality of ultrasound transducer element such as 32 or more ultrasound transducer elements. Each ultrasound transducer element must be provided with an electrical conductor for providing and receiving electrical signals.

Performing the required electrical wiring is however a difficult and time-consuming task. This is one of the reasons why it is complex and expensive to manufacture a curvilinear ultrasound transducer.

Ultrasound endoscopes are therefore significantly more expensive than regular endoscopes.

Availability of endoscopic ultrasound is therefore limited for some patient groups.

Furthermore, endoscopes are difficult to clean and there exist the risk of cross-contamination, i.e. that a pathological condition is transferred between patients as result of improper cleaning.

The most secure solution has been the introduction of single-use endoscopes. A single use endoscope eliminates any risk of cross-contamination.

The cost of an ultrasound transducer has however made it difficult to provide single use ultrasound endoscopes. This is problematic since the provision of an ultrasound probe at the distal end of the endoscope makes the endoscope even harder to clean.

Thus, it remains a problem to provide an improved ultrasound transducer.

### Summary

According to a first aspect, the present disclosure relates to an ultrasound transducer (100) for receiving and transmitting ultrasound comprising a convex ultrasound transducer array comprising a plurality of ultrasound transducer elements (180), an electrical circuit for receiving and providing electrical signals to the plurality of ultrasound transducer elements (180), and a support structure (101) having a convex upper surface (102) facing the plurality of ultrasound transducer elements (180) and extending in a direction parallel to a first axis (191), wherein the electrical circuit comprises
a flexible base section (103) comprising a plurality of first electrical contacts (110-122) each first electrical contact (110-122) being connected to an ultrasound transducer element for providing and receiving electrical signals to / from the ultrasound transducer element and extending along a central axis (193) being parallel to the first axis (191), the flexible base section (103) being arranged on the convex upper surface (102) of the support structure (101),
a plurality of first flexible electrical conductors (150-162) each first flexible electrical conductor being electrically connected to a first electrical contact (110-122),
a contact section comprising a plurality of second electrical contacts each second electrical contact being electrically connected to a first electrical conductor (150-162) and further electrical connected or connectable to a second electrical conductor or a transducer port of an ultrasound processing apparatus,
wherein the plurality of first flexible electrical conductors (150-162) are bent around the support structure (101) at a first curve section (140) and at a second curve section (141), the first curve section (140) being separate from the second curve section (141).

Consequently, by using an electrical circuit provided with a flexible base section having a plurality of electrical contacts, the plurality of ultrasound transducer elements may be provided with an electrical connection in a single process step. This makes it simple to manufacture. Additionally, by providing the electrical circuit with a plurality of flexible electrical conductors bent around the support structure at a first curve section and second curve section, a single electrical circuit may be used to transport the electrical signal to and from the plurality of electrical contacts as the electric circuit may conform to the curvature of the convex upper surface.

The flexible base section and / or the plurality of first flexible conductors may be a flexible printed circuit. The flexible printed circuit may be made using Photolithography. An adhesive may be provided between the lower side of the ultrasound transducer array and the flexible base section. Correspondingly, an adhesive may be provided between the flexible base section and the convex upper surface of the support structure. After attachment to the plurality of ultrasound transducer elements the flexibility of the flexible base section is limited by the flexibility of the ultrasound transducer array.

The ultrasound transducer elements may be made from a piezo electric block. The piezoelectric block may be made of any kind of piezoelectric material such as lead zirconate titanate (PZT), Quartz, Barium titanate, Poly(vinylidene fluoride) (PVDF), or a piezoelectric composite material.

The ultrasound transducer may be provided with one or more acoustic matching layers configured to provide an acoustic impedance gradient between the acoustic impedance of the tissue and the acoustic impedance of the ultrasound transducer elements. The acoustic matching layer may be made of epoxy, polyurethane, or polystyrene.

The ultrasound transducer may be configured to generate ultrasound with a centre frequency between 1 MHz and 15 MHz, or 2MHz and 8MHz.

The plurality of ultrasound transducer elements may be arranged in a common reference plane and configured to image a single image plane.

The plurality of ultrasound transducer elements may have been prepoled and metalized before being attached to flexible base section. As an example, one or two layers of a nickel-chromium alloy may be applied to the plurality of ultrasound transducer elements. Alternatively / additionally one or two layers of Au may be applied. The layer may preferably be thin layers having a thickness of 10-20nm. The layers may improve the piezoelectric properties of the piezoelectric block.

The support structure is preferably made of a material configured to absorb ultrasound energy. The material may be a composite material. As an example, the material may be an epoxy mixed with tungsten powder.

The flexible base section, the plurality of first flexible electrical conductors, and the contact section of the electrical circuit may be produced as a single integral element.

The ultrasound transducer may further comprise an acoustic lens for focusing the ultrasound energy. The acoustic lens may be configured to focus the ultrasound energy in the image plane.

The plurality of first flexible electrical conductors may be bent around the support structure at more curve sections than the first curve section and the second curve section. As an example, the plurality of first flexible electrical conductors may be bent around the support structure at least at three, four, six, or eight curve sections.

In some embodiments, the support structure (101) has a first side surface (104) arranged in a plane being perpendicular to the first axis (191), the first side surface (104) being connected to the convex upper surface (102) and wherein the first curve section (140) is arranged along the connection between the convex upper surface (102) and the first side surface (104).

In some embodiments, the second curve section (141) is arranged along the connection between the convex upper surface and the first side surface (104) and divided from the first curve section (140) by a gap (142).

Consequently, it may become possible to guide a significant number (or all) of the flexible electrical conductors away from the convex upper surface of the support structure in a simple and compact manner.

In some embodiments, the support structure comprises a concave lower surface (106) opposite to the convex upper surface (102), the first side surface (104) is connected to the concave lower surface (106), the gap (142) extends along the entire first side (104) of the support structure (101), and wherein the plurality of the first flexible electrical conductors (150-162) are further bent around the support structure at a third curve section (143) and at a fourth curve section (144), the third curve section (143) being separate from the fourth curve section (144) by the gap (142), and wherein the third curve section (143) and the fourth curve section (144) are arranged along the connection between the first side surface (104) and the concave lower surface (106).

Consequently, the concave lower surface may be utilized to provide space for directing the plurality of first flexible electrical conductors away from the ultrasound transducer elements. This may reduce the amount of space required by the electric circuit.

In some embodiments, the plurality of first flexible electrical conductors comprises a first group of flexible electrical conductors (150-156) and a second group of flexible electrical conductors (157-162), the support structure (101) has a second side surface (105) opposite to the first side surface (104), the second side surface (105) arranged in a plane being perpendicular to the first axis (191), the second side surface (105) being connected to the convex upper surface (102), the second curve section (141) is arranged along the connection between the convex upper surface (102) and the second side surface (105), and wherein the first group of flexible electrical conductors (150-156) are bent around the first curve section (140) and the second group of flexible electrical conductors (157-162) are bent around the second curve section (141).

Consequently, it may become possible to utilize space on both sides of the support structure for the plurality of first electrical conductors.

In some embodiments, the first group of flexible electrical conductors (150-156) are arranged in a first plane along a part of their length, the second group of flexible electrical conductors (157-162) are arranged in a second plane along a part of their length, where the first plane is arranged above the second plane.

Consequently, by packing the flexible electrical conductors vertically, the required space in the width may be reduced. This may be advantageous as flexible electrical may be created very thin, whereby the increased space required in the height may handle relatively easily.

In some embodiments, the first plane is parallel with the second plane.

In some embodiments, each of the plurality of second electrical contacts are connected to a second electrical conductor.

The first electrical circuit will typically be manufactured as a flat structure and subsequently folded into the form required by the ultrasound transducer. Thus, by connecting the second electrical contact to a second electrical conductor the folding task may be simplified.

In some embodiments, for each of the plurality of second electrical contacts the second electrical conductor is a coaxial cable permanently connected to the second electrical contact.

Consequently, by using coaxial cables in connection with a flexible electrical circuit the connections between the coaxial cables and the ultrasound transducer elements may be done at a distance from the ultrasound transducer elements, where there is more space.

In some embodiments, for each of the plurality of second electrical contacts the second electrical conductor is a second flexible electrical conductor permanently connected the second electrical contact.

Consequently, by also using flexible electrical conductors to transfer electrical signal further away from the transducer elements, the space requirements of the entire electrical connection may be kept low.

In some embodiments, each of the plurality of second electrical contacts are configured to be detachable connectable to a second electrical conductor or a transducer port of an ultrasound processing apparatus.

In some embodiments, the flexible base section is a single flexible printed circuit.

Consequently, both the manufacturing of the base section and the subsequent attachment to the ultrasound transducer elements may be simplified.

In some embodiments, the flexible base section and the plurality of first flexible electrical conductors is a single flexible printed circuit.

Consequently, both the manufacturing of the base section and the subsequent attachment to the ultrasound transducer elements may be simplified.

According to a second aspect, the present disclosure relates to a printed flexible electrical circuit for receiving and providing electrical signals to the plurality of ultrasound transducer elements, the printed flexible electrical circuit comprising,
a flexible base section (103) comprising a plurality of first electrical contacts (110-122) each first electrical contact (110-122) being connectable to an ultrasound transducer element for providing and receiving electrical signals to the ultrasound transducer element and extending along a central axis (193) being parallel to a first axis (191),
a plurality of first flexible electrical conductors (150-162) each first flexible electrical conductor being electrically connected to a first electrical contact (110-122),
a contact section comprising a plurality of second electrical contacts each second electrical contact being electrically connected to a first electrical conductor (150-162) and further electrical connected or connectable to a second electrical conductor or a transducer port of an ultrasound processing apparatus,
wherein the plurality of first flexible electrical conductors (150-162) is bendable around a support structure (101) at a first curve section (140) and at a second curve section (141), the first curve section (140) being separate from the second curve section (141).

Consequently, by providing an electrical circuit with a flexible base section having a plurality of electrical contacts, a plurality of ultrasound transducer elements may be provided with an electrical connection in a single process step. This makes it simple to manufacture. Additionally, by providing the electrical circuit with a plurality of flexible electrical conductors bendable around a support structure at a first curve section and second curve section, a single electrical circuit may be used to transport the electrical signal to and from the plurality of electrical contacts.

According to a third aspect, the present disclosure relates to an endoscope comprising a handle, an insertion tube, an image capturing device, a first flexible cable and an ultrasound transducer as disclosed in relation to the first aspect, the handle being attached to a proximal end of the insertion tube, the image capturing device and the ultrasound transducer being attached to a distal end of the insertion tube, and the first flexible cable is extending from the handle and being electrically connected to the plurality of ultrasound transducer elements via the electrical circuit and wherein the first flexible cable is connectable to an ultrasound processing apparatus and configured to provide the ultrasound processing apparatus with electrical signals generate by the plurality of ultrasound transducer elements and receive electrical signals generated by the ultrasound processing apparatus for the plurality of ultrasound transducer elements.

Ultrasound processing apparatuses are well-known in the art. Ultrasound processing apparatuses are configured to generate the electrical signals needed by the ultrasound transducer elements to generate the ultrasound waves. The electrical signal should have a centre frequency substantially matching the centre frequency of the ultrasound transducer elements. Furthermore, the signal should have a length and amplitude securing that a sufficient SNR is achieved. Furthermore, the ultrasound processing apparatus is configured to generate the ultrasound images by processing the signals generated by the ultrasound transducer elements. The ultrasound imaging may be done by using ultrasound beamforming techniques such as delay and sum beamforming in receive. In transmit, the ultrasound energy may be focused in a particular point by applying a suitable delay to the individual ultrasound transducer elements. Alternatively, a plane wave or a diverging ultrasound wave may be used in transmit.

The ultrasound processing apparatus may be adapted to cause a display to display a live representation of the electrical signals received from the ultrasound transducer. The ultrasound processing apparatus may be couplable to a display. The ultrasound processing apparatus may comprise a display for displaying a live representation of the electrical signals received from the ultrasound transducer and/or the image capturing device.

The ultrasound processing apparatus may be a portable ultrasound processing apparatus.

In some embodiments, the first flexible cable is further configured to receive electrical signals from the image capturing device and provide the received electrical signals to the ultrasound processing apparatus.

Consequently, by using a single flexible cable to transfer signals from both the ultrasound transducer and the image capturing device it may becomes simpler for user to operate the endoscope.

The ultrasound processing apparatus may comprise one or more processing units configured to process both the signals received from the ultrasound transducer and the signals received from the image capturing device.

Alternatively, the ultrasound processing apparatus may comprise a first set of one or more processing units configured to process the signals received from the ultrasound transducer and a second set of one or more processing units configured to process the signals received form the image capturing device. The first set and second set of one or more processing units may be provided in the same housing or in separate housings where the separate housings are communicatively coupled.

In some embodiments, the endoscope further comprises a second flexible cable configured to receive electrical signals from the image capturing device and provide the received electrical signals to an video processing apparatus.

Consequently, it may become simpler to connect the endoscope to a separate ultrasound processing apparatus and video processing apparatus.

The video processing apparatus may be adapted to cause a display to display a live representation of the electrical signals received from image capturing device. The video processing apparatus may be couplable to a display. The video processing apparatus may comprise a display for displaying a live representation of the electrical signals received from the image capturing device.

The video processing apparatus may be a portable video processing apparatus.

In some embodiments, the first flexible cable and the second flexible cable are connected along a first part of their length and disconnected along a second part of their length.

Consequently, it may both become simpler for the user to operate the endoscope and connect the cables to a separate ultrasound processing apparatus and video processing apparatus.

The first flexible cable may be attached to the second cable using an adhesive. Additionally / alternatively an outer cable may be arranged around the first flexible cable and the second flexible cable along the first part of their length.

According to a fourth aspect, the present disclosure relates to a system comprising an endoscope as disclosed in relation to the third aspect of the present disclosure and an ultrasound processing apparatus, wherein the first flexible cable is connectable to the ultrasound processing apparatus.

In some embodiments, the first flexible cable is further configured to receive electrical signals from the image capturing device and provide the received electrical signals to the ultrasound processing apparatus, and wherein the ultrasound processing apparatus comprise one or more processing units configured to process both the signals received from the ultrasound transducer and the signals received from the image capturing device.

In some embodiments, the system further comprises an video processing apparatus and wherein the endoscope further comprises a second flexible cable configured to receive electrical signals from the image capturing device and provide the received electrical signals to the video processing apparatus.

In some embodiments, the first flexible cable and the second flexible cable are connected along a first part of their length and disconnected along a second part of their length.

The different aspects of the present disclosure can be implemented in different ways including as an ultrasound transducer, a printed flexible electrical circuit, an endoscope comprising an ultrasound transducer, and an endoscope system described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1a-b show schematically a support structure for an ultrasound transducer according to an embodiment of the disclosure.
Fig. 2a-c show schematically a support structure and an electrical circuit for an ultrasound transducer according to an embodiment of the disclosure.
Fig. 3a-c show schematically a support structure and an electrical circuit for an ultrasound transducer according to an embodiment of the disclosure.
Fig. 4a-b show schematically a support structure and an electrical circuit for an ultrasound transducer according to an embodiment of the disclosure.
Fig. 5 shows schematically an ultrasound transducer according to an embodiment of the disclosure.
Fig. 6 shows schematically a system according to an embodiment of the disclosure.
Fig. 7 shows schematically a system according to an embodiment of the disclosure.

### Detailed description

In the following description, reference is made to the accompanying figures, which show by way of illustration how the embodiments of the present disclosure may be practiced.

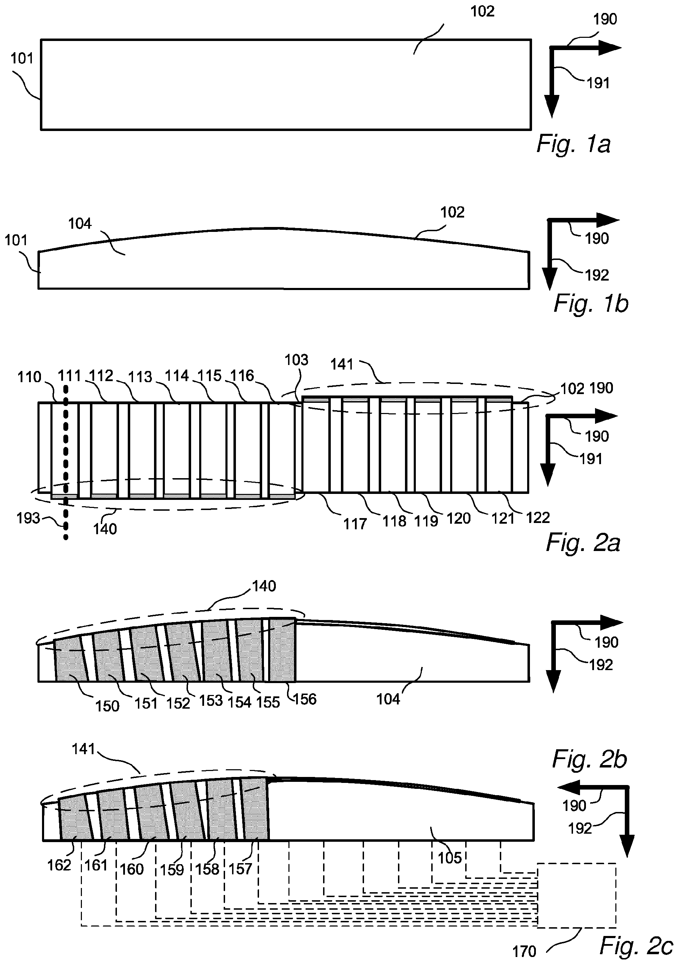

Fig. 1a-b show schematically a support structure (101) for an ultrasound transducer according to an embodiment of the disclosure, where Fig. 1a shows a top view and Fig. 1b shows a side view. The support structure (101) has a convex upper surface (102) for facing a plurality of ultrasound transducer elements. The convex upper surface (102) extends in a direction parallel to a first axis (191). There is further shown a second axis (190) and a third axis (192). The second axis (190) being perpendicular to the first axis (191), and the third axis (192) being perpendicular to both the first axis (191) and the second axis (190). In addition to supporting the ultrasound transducer elements, the support structure may assist in absorbing ultrasound energy. Thus, the support structure (101) is preferably made of a material configured to absorb ultrasound energy. The material may be a composite material. The material may be an epoxy mixed with tungsten powder.

Fig. 2a-c show schematically a support structure (101) and an electrical circuit for an ultrasound transducer according to an embodiment of the disclosure, where Fig. 2a shows a top view, Fig. 2b shows a first side view, and Fig. 2c shows a second side view. The support structure (101) corresponds to the support structure (101) shown in Fig. 1a-b. The electrical circuit comprises a flexible base section (103) comprising a plurality of first electrical contacts (110-122) each first electrical contact (110-122) being connectable to an ultrasound transducer element for providing and receiving electrical signals to / from the ultrasound transducer element. Each of the first electrical contacts (110-122) extends along a central axis (193) being parallel to the first axis (191). For simplicity only the central axis (193) for the first electrical contact 110 is shown. The flexible base section (103) is arranged on the convex upper surface (102) of the support structure (101). The first electrical contacts (110-122) should be made of a material having a high electrical conductivity such as Cu, Al, Ag, or Au. The area of the flexible base section (103) between the plurality of first electrical contacts (110-122) should be electrically isolating to prevent crosstalk between the ultrasound transducer elements. Correspondingly, the flexible base section (103) is preferably provided with one or more electrically isolating lower layers. For simplicity only 13 first electrical contacts (110-122) are shown. However, in other embodiments there may be at least 16, 32 or 64 first electrical contacts. The electrical circuit further comprises a plurality of first flexible electrical conductors (150-162) each first flexible electrical conductor being electrically connected to a first electrical contact (110-122). The plurality of first flexible electrical conductors (150-162) are responsible for transferring electrical signals from the plurality of first electrical contacts (110-122) towards an ultrasound processing apparatus and transferring electrical signals from an ultrasound processing apparatus towards the plurality of first electrical contacts (110-122). The plurality of first flexible electrical conductors (150-162) should be made of a material having a high electrical conductivity such as Cu, Al, Ag, or Au. The area between the plurality of first flexible electrical conductors (150-162) should be electrically isolating to prevent cross-talk between the ultrasound transducer elements. Correspondingly, the plurality of first flexible electrical conductors (150-162) are preferably provided with one or more electrically isolating upper layers and one or more electrically isolating lower layers. The plurality of first flexible electrical conductors (150-162) are bent around the support structure (101) at a first curve section (140) and at a second curve section (141), the first curve section (140) being separate from the second curve section (141). The plurality of first flexible electrical conductors comprises a first group of flexible electrical conductors (150-156) and a second group of flexible electrical conductors (157-162). The support structure (101) has a first side surface (104) being arranged in a plane (not shown) being perpendicular to the first axis (191), the first side surface (104) being connected to the convex upper surface (102) and wherein the first curve section (140) is arranged along the connection between the convex upper surface (102) and the first side surface (104). The support structure (101) has a second side surface (105) opposite to the first side surface (104), the second side surface (105) being arranged in a plane (not shown) being perpendicular to the first axis (191), the second side surface (105) being connected to the convex upper surface (102), the second curve section (141) is arranged along the connection between the convex upper surface (102) and the second side surface (105). The first group of flexible electrical conductors (150-156) are bent around the first curve section (140) and the second group of flexible electrical conductors (157-162) are bent around the second curve section (141). Only the first part of the plurality of first electrical conductors is shown in Fig. 2a-b. In Fig. 2c are the remaining part of the first electrical conductors are schematically illustrated by dashed lines. The electrical circuit further comprises a contact section (170) comprising a plurality of second electrical contacts each second electrical contact being electrically connected to a first electrical conductor (150-162) and further electrical connected or connectable to a second electrical conductor or a transducer port of an ultrasound system.

In some embodiments, the contact section (170) is the ultrasound connector that is inserted into the transducer port of an ultrasound processing apparatus and the first electrical conductors (150-162) are responsible for the entire electrical coupling between the plurality of first electrical contacts (110-122) and the ultrasound connector.

In other embodiments, each of the plurality of second electrical contacts of the contact section (170) are connected to a second electrical conductor and the first electrical conductors (150-162) are only responsible for part of the electrical coupling between the plurality of first electrical contacts (110-122) and the ultrasound connector. The second electrical conductor may be a coaxial cable or a printed flexible electrical conductor.

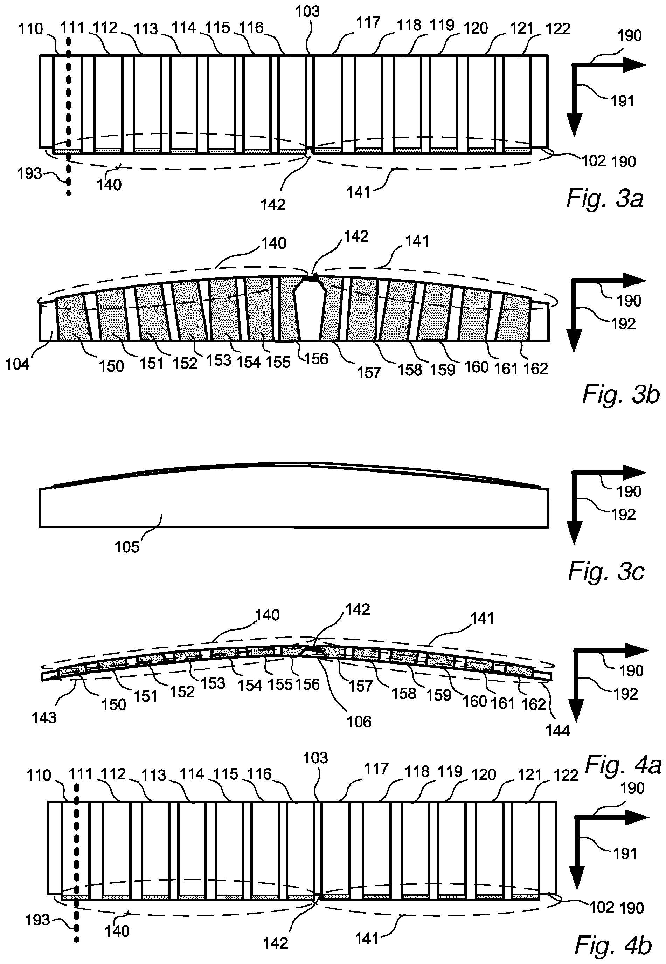

Fig. 3a-c show schematically a support structure (101) and an electrical circuit for an ultrasound transducer according to an embodiment of the disclosure, where Fig. 3a shows a top view, Fig. 3b shows a first side view, and Fig. 3c shows a second side view. The support structure (101) corresponds to the support structure (101) shown in Fig. 1a-b. The electrical circuit comprises a flexible base section (103) comprising a plurality of first electrical contacts (110-122) each first electrical contact (110-122) being connectable to an ultrasound transducer element for providing and receiving electrical signals to / from the ultrasound transducer element. Each of the first electrical contacts (110-122) extends along a central axis (193) being parallel to the first axis (191). For simplicity only the central axis (193) for the first electrical contact 110 is shown. The flexible base section (103) is arranged on the convex upper surface (102) of the support structure (101). The electrical circuit further comprises a plurality of first flexible electrical conductors (150-162) each first flexible electrical conductor being electrically connected to a first electrical contact (110-122). The plurality of first flexible electrical conductors (150-162) are responsible for transferring electrical signals from the plurality of first electrical contacts (110-122) towards an ultrasound processing apparatus and transferring electrical signals from an ultrasound processing apparatus towards the plurality of first electrical contacts (110-122). The support structure (101) has a first side surface (104) arranged in a plane (not shown) being perpendicular to the first axis (191), the first side surface (104) being connected to the convex upper surface (102). The plurality of first flexible electrical conductors (150-162) are bent around the support structure (101) at a first curve section (140) and at a second curve section (141), the first curve section (140) being separate from the second curve section (141). The first curve section (140) and the second curve section (141) are arranged along the connection between the convex upper surface (102) and the first side surface (104) and divided by a gap (142). Only the first part of the plurality of first electrical conductors is shown in Fig. 3a-c. The electrical circuit may further comprise a contact section (170) comprising a plurality of second electrical contacts each second electrical contact being electrically connected to a first electrical conductor (150-162) and further electrical connected or connectable to a second electrical conductor or a transducer port of an ultrasound system.

Fig. 4a-b show schematically a support structure (101) and an electrical circuit for an ultrasound transducer according to an embodiment of the disclosure, where Fig. 4a shows a top view and Fig. 4b shows a side view. The embodiment shown in Fig. 4a-b corresponds to the embodiment shown in Fig. 3a-c with the difference that the support structure (101) comprises a concave lower surface (106) opposite to the convex upper surface (102), the first side surface (104) is connected to the concave lower surface (106) and the gap (142) extends along the entire first side (104) of the support structure (101). The plurality of first flexible electrical conductors (150-162) are further bent around the support structure at a third curve section (143) and at a fourth curve section (144), the third curve section (143) being separate from the fourth curve section (144) by the gap (142), and wherein the third curve section (143) and the fourth curve section (144) are arranged along the connection between the first side surface (104) and the concave lower surface (106). Consequently, the concave lower surface may be utilized to provide space for directing the plurality of first flexible electrical conductors away from the ultrasound transducer elements. This may reduce the amount of space required by the electric circuit.

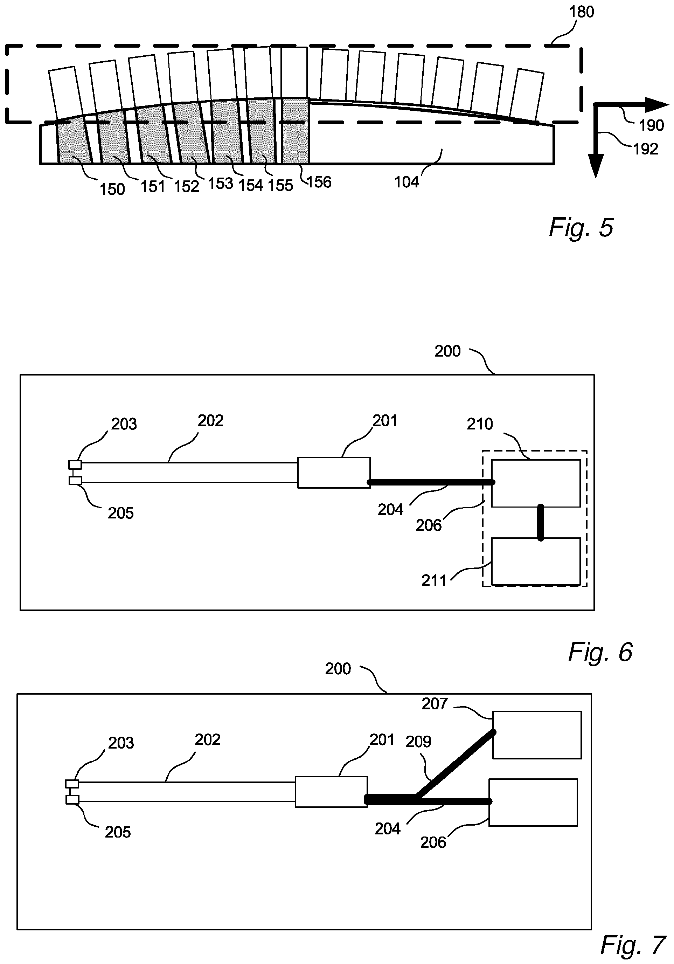

Fig. 5 shows schematically an ultrasound transducer according to an embodiment of the disclosure. The ultrasound transducer comprises a support structure (101) and an electrical circuit corresponding to the support structure and the electrical circuit disclosed in relation to Fig. 2a-c. Furthermore, the ultrasound transducer comprises a convex ultrasound transducer array comprising a plurality of ultrasound transducer elements (180), where each first electrical contact (110-122) is connected to an ultrasound transducer element for providing and receiving electrical signals to / from the ultrasound transducer element.

Fig. 6 shows schematically a system (200) comprising an endoscope and an ultrasound processing apparatus (206) according to an embodiment of the disclosure. The endoscope comprises a handle (201), an insertion tube (202), an image capturing device (203), a first flexible cable (204) and an ultrasound transducer (205). The ultrasound transducer may be an ultrasound transducer as disclosed in Figs. 1-5. The handle (201) is attached to a proximal end of the insertion tube (202) and the image capturing device (203) and the ultrasound transducer (205) are attached to a distal end of the insertion tube (202). The first flexible cable (204) is extending from the handle (201) and is electrically connected to the ultrasound transducer (205) and wherein the first flexible cable is connected to the ultrasound processing apparatus (206) and configured to provide the ultrasound processing apparatus (206) with electrical signals generate by the ultrasound transducer (205) and receive electrical signals generated by the ultrasound processing apparatus (206) for the ultrasound transducer (205). In this embodiment, the first flexible cable (204) is further configured to receive electrical signals from the image capturing device (203) and provide the received electrical signals to the ultrasound processing apparatus (206). The ultrasound processing apparatus (206) comprises a first processing unit (210) configured to process electrical signals generate by the ultrasound transducer (205) and a second processing unit (211) configured to process image signals recorded by the image capturing device (203).

Fig. 7 shows schematically a system (200) comprising an endoscope an ultrasound processing apparatus (206), and an imaging unit (207) according to an embodiment of the disclosure. The endoscope comprises a handle (201), an insertion tube (202), an image capturing device (203), a first flexible cable (204), a second flexible cable (209) and an ultrasound transducer (205). The ultrasound transducer (205) may be an ultrasound transducer as disclosed in Figs. 1-5. The handle (201) is attached to a proximal end of the insertion tube (202) and the image capturing device (203) and the ultrasound transducer (205) are attached to a distal end of the insertion tube (202). The first flexible cable (204) is extending from the handle (201) and is electrically connected to a plurality of ultrasound transducer elements via an electrical circuit and wherein the first flexible cable is connected to the ultrasound processing apparatus (206) and configured to provide the ultrasound processing apparatus (206) with electrical signals generate by the ultrasound transducer (205) and receive electrical signals generated by the ultrasound processing apparatus (206) for the ultrasound transducer (205). In this embodiment, the second flexible cable (209) is configured to receive electrical signals from the image capturing device (203) and provide the received electrical signals to the imaging unit (209).

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An ultrasound transducer (100) for receiving and transmitting ultrasound comprising a convex ultrasound transducer array comprising a plurality of ultrasound transducer elements (180), an electrical circuit for receiving and providing electrical signals to the plurality of ultrasound transducer elements (180), and a support structure (101) having a convex upper surface (102) facing the plurality of ultrasound transducer elements (180) and extending in a direction parallel to a first axis (191), wherein the electrical circuit comprises
a flexible base section (103) comprising a plurality of first electrical contacts (110-122) each first electrical contact (110-122) being connected to an ultrasound transducer element for providing and receiving electrical signals to / from the ultrasound transducer element and extending along a central axis (193) being parallel to the first axis (191), the flexible base section (103) being arranged on the convex upper surface (102) of the support structure (101),
a plurality of first flexible electrical conductors (150-162) each first flexible electrical conductor being electrically connected to a first electrical contact (110-122),
a contact section comprising a plurality of second electrical contacts each second electrical contact being electrically connected to a first electrical conductor (150-162) and further electrically connected or connectable to a second electrical conductor or a transducer port of an ultrasound processing apparatus,
wherein the plurality of first flexible electrical conductors (150-162) are bent around the support structure (101) at a first curve section (140) and at a second curve section (141), the first curve section (140) being separate from the second curve section (141).

2. An ultrasound transducer according to claim 1, wherein the support structure (101) has a first side surface (104) arranged in a plane being perpendicular to the first axis (191), the first side surface (104) being connected to the convex upper surface (102) and wherein the first curve section (140) is arranged along the connection between the convex upper surface (102) and the first side surface (104).

3. An ultrasound transducer according to claim 2, wherein the second curve section (141) is arranged along the connection between the convex upper surface and the first side surface (104) and divided from the first curve section (140) by a gap (142).

4. An ultrasound transducer according to claim 3, wherein the support structure comprises a concave lower surface (106) opposite to the convex upper surface (102), the first side surface (104) is connected to the concave lower surface (106), the gap (142) extends along the entire first side (104) of the support structure (101), and wherein the plurality of the first flexible electrical conductors (150-162) are further bent around the support structure at a third curve section (143) and at a fourth curve section (144), the third curve section (143) being separate from the fourth curve section (144) by the gap (142), and wherein the third curve section (143) and the fourth curve section (144) are arranged along the connection between the first side surface (104) and the concave lower surface (106).

5. An ultrasound transducer according to claim 2, wherein the plurality of first flexible electrical conductors comprises a first group of flexible electrical conductors (150-156) and a second group of flexible electrical conductors (157-162), the support structure (101) has a second side surface (105) opposite to the first side surface (104), the second side surface (105) arranged in a plane being perpendicular to the first axis (191), the second side surface (105) being connected to the convex upper surface (102), the second curve section (141) is arranged along the connection between the convex upper surface (102) and the second side surface (105), and wherein the first group of flexible electrical conductors (150-156) are bent around the first curve section (140) and the second group of flexible electrical conductors (157-162) are bent around the second curve section (141).

6. An ultrasound transducer according to claim 5, wherein the first group of flexible electrical conductors (150-156) are arranged in a first plane along a part of their length, the second group of flexible electrical conductors (157-162) are arranged in a second plane along a part of their length, where the first plane is arranged above the second plane.

7. An ultrasound transducer according to any one of claim 1 to 6,
wherein each of the plurality of second electrical contacts are connected to a second electrical conductor.

8. An ultrasound transducer according to claim 7, wherein for each of the plurality of second electrical contacts the second electrical conductor is a coaxial cable permanently connected the second electrical contact.

9. An ultrasound transducer according to any one of claim 1 to 6,
wherein each of the plurality of second electrical contacts are configured to be detachable connectable to a second electrical conductor or a transducer port of an ultrasound processing apparatus.

10. An ultrasound transducer according to any one of claims 1 to 9,
wherein the flexible base section and the plurality of first flexible electrical conductors is a single flexible printed circuit.

11. A printed flexible electrical circuit for receiving and providing electrical signals to the plurality of ultrasound transducer elements, the printed flexible electrical circuit comprising,
a flexible base section (103) comprising a plurality of first electrical contacts (110-122) each first electrical contact (110-122) being connectable to an ultrasound transducer element for providing and receiving electrical signals to the ultrasound transducer element and extending along a central axis (193) being parallel to a first axis (191),
a plurality of first flexible electrical conductors (150-162) each first flexible electrical conductor being electrically connected to a first electrical contact (110-122),
a contact section comprising a plurality of second electrical contacts each second electrical contact being electrically connected to a first electrical conductor (150-162) and further electrical connected or connectable to a second electrical conductor or a transducer port of an ultrasound processing apparatus,
wherein the plurality of first flexible electrical conductors (150-162) is bendable around a support structure (101) at a first curve section (140) and at a second curve section (141), the first curve section (140) being separate from the second curve section (141).

12. An endoscope comprising a handle, an insertion tube, an image capturing device, a first flexible cable and an ultrasound transducer according to any one of claims 1 to 11, the handle being attached to a proximal end of the insertion tube, the image capturing device and the ultrasound transducer being attached to a distal end of the insertion tube, and the first flexible cable is extending from the handle and being electrically connected to the plurality of ultrasound transducer elements via the electrical circuit and wherein the first flexible cable is connectable to an ultrasound processing apparatus and configured to provide the ultrasound processing apparatus with electrical signals generate by the plurality of ultrasound transducer elements and receive electrical signals generated by the ultrasound processing apparatus for the plurality of ultrasound transducer elements.

13. A system comprising an endoscope according to claim 12 and an ultrasound processing apparatus, wherein the first flexible cable is connectable to the ultrasound processing apparatus.

14. A system according to claim 13, wherein the first flexible cable is further configured to receive electrical signals from the image capturing device and provide the received electrical signals to the ultrasound processing apparatus, and wherein the ultrasound processing apparatus comprise one or more processing units configured to process both the signals received from the ultrasound transducer and the signals received from the image capturing device.

15. A system according to claim 13, wherein the system further comprises an video processing apparatus and wherein the endoscope further comprises a second flexible cable configured to receive electrical signals from the image capturing device and provide the received electrical signals to the video processing apparatus.
